# EUROPÄISCHE PATENTSCHRIFT

(11) **EP 0 696 569 B1**
(45) Veröffentlichungstag und Bekanntmachung des Hinweises auf die Patenterteilung: **28.10.1998**
(21) Anmeldenummer: 95111991.6
(22) Anmeldetag: 31.07.1995
(51) Int. Cl.: C07C 205/06, C07C 201/08

(54) **Verfahren zur Herstellung von Dinitrotoluol**
Process for the preparation of dinitrotoluene
Procédé de préparation de dinitrotoluène

(30) Priorität: 11.08.1994 DE 4428461
(43) Veröffentlichungstag der Anmeldung: 14.02.1996
(73) Patentinhaber: BAYER AG, 51368 Leverkusen (DE)
(72) Erfinder: Klingler, Uwe, D-41539 Dormagen (DE); Schieb, Thomas, Dr., D-51503 Rösrath (DE); Wastian, Dietmar, D-41542 Dormagen (DE); Wiechers, Gerhard, Dr., D-51381 Leverkusen (DE); Zimmermann, Jürgen, Dr., Walnut Creek, CA 94598 (US)

(56) Entgegenhaltungen:
- EP-A- 0 155 586
- EP-A- 0 597 361
- DE-A- 2 655 197

## Beschreibung

Die vorliegende Erfindung betrifft die Herstellung von Dinitrotoluol durch Nitrierung von Toluol mit Nitriersäure unter adiabatischen Bedingungen.

Es ist bekannt, Toluol auf adiabatischem Wege in Dinitrotoluol (DNT) zu überführen (EP-A 597 361). Hierbei wird Toluol mit mindestens 2 Äquivalenten einer Nitriersäure bestimmter Zusammensetzung auf adiabatischem Wege umgesetzt, wobei eine Endtemperatur von über 120°C erreicht wird. Nach Trennung der Phasen bei dieser Temperatur wird die Säurephase einer Aufkonzentrierung (Flashverdampfung im Vakuum) zugeführt, wobei der Wärmeinhalt der Säure zur Aufkonzentrierung genutzt wird. Die aufkonzentrierte Säure wird mit Salpetersäure aufgestockt und in den Prozeß zurückgeführt.

Bei diesem Verfahren ergibt sich die Schwierigkeit, daß mit dem abdestillierenden Wasser bei der Flashverdampfung ein gewisser, in der Säure gelöster Teil an DNT mit übergeht, welcher unter den Kondensationsbedingungen des Wassers fest wird (Festpunkt des Isomerengemisches ca. 55°C) und den Wärmetauscher belegt. Für derartige Verhältnisse bieten sich zwei Lösungsmöglichkeiten an.

Zum einen können in derartigen Fällen getaktete Wärmetauscher eingesetzt werden. Diese werden jeweils wechselseitig betrieben und nach Belegung in der Ruhephase aufgeschmolzen. Problematisch bei der Belegung der Austauscherflächen ist die dadurch rasch nachlassende Kühlleistung. Dementsprechend muß häufig getaktet werden. Das Abschmelzen des jeweils ruhenden Kondensators (aufheizen und wieder abkühlen) erfordert zusätzliche Energie.

Als weitere Möglichkeit zur Kondensation von feststoffbildenden Brüden wird der Misch- oder Einspritzkondensator beschrieben (R.A. Vauck, H.A. Müller, Grundoperationen chemischer Verfahrenstechnik, 5. Auflage, VEB Leipzig 1962, S. 447). Darunter wird das Einbringen der Brüden in einen Sprühstrahl aus kaltem Wasser verstanden, wobei für den obengenannten Fall DNT in feinverteilter Form als Feststoff abgeschieden wird. Dabei kann sowohl im Gleich- als auch im Gegenstrom gearbeitet werden. Aufgrund der hierfür notwendigen großen Wassermengen wird das Wasser sinnvollerweise im Kreis geführt und im Rückführungszweig gekühlt. Zur Abtrennung z.B. des DNTs wird ein Teilstrom ausgeschleust. Hierbei besteht allerdings die Gefahr der Verstopfung von Leitungen und Düsen durch organische Komponenten mit niedrigem Schmelzpunkt, die zum Verkleben neigen. Die Reingewinnung des DNTs ist außerdem mit einem Energieeintrag verbunden, da erst aufgescholzen und dann flüssig/flüssig getrennt werden muß.

Eine wesentlich einfachere und elegantere Lösung des Problems besteht in der isothermen zweistufigen Herstellung von Dinitrotoluol (Ullmann Encyklopädie der technischen Chemie, 4. Auflage, Band 17, Seite 392, Verlag Chemie, Weinheim 1979). Hierbei wird zunächst ein Gemisch der isomeren Mononitrotoluole (MNT) hergestellt, welches in einem zweiten separaten Verfahrensschritt zu Dinitrotoluolen umgesetzt wird. Das Problem des belegten Wärmetauschers bei der Aufkonzentrierung der Absäure im Vakuum wird durch Einspritzen von MNT aus der ersten Stufe in die Brüden behoben (DE-A 34 09 719). Das eingespritzte MNT bewirkt eine Schmelzpunktserniedrigung des DNTs und sorgt somit dafür, daß die Brüden auch unter den Kondensationsbedingungen des Wassers flüssig bleiben. Die über Phasentrennung isolierte organische Phase wird in die Reaktion zurückgeführt.

Diese elegante Lösung kann beim Verfahren der einstufigen, adiabatischen Dinitrierung von Toluol (EP-A 597 361) nicht zur Anwendung gelangen, da im Prozeß kein frei verfügbares MNT vorliegt. Dieses tritt lediglich als nicht isolierbares Zwischenprodukt in der Reaktion auf.

Aufgabe war es, ein kontinuierliches, adiabatisches Verfahren zur Herstellung von Dinitrotoluol zur Verfügung zu stellen, bei dem die bisher auftretenden Probleme bzw. die aufwendigen Verfahrensschritte und zusätzlicher Energieaufwand vermieden werden.

Gegenstand der Erfindung ist ein Verfahren zur kontinuierlichen Herstellung von Dinitrotoluol-Isomerengemischen durch Nitrierung von Toluol, welches dadurch gekennzeichnet ist, daß man Toluol einstufig in einem kontinuierlich betriebenen Reaktor unter Verwendung einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu über 70 % aus Dinitrotoluol-Isomeren und zum Rest aus Nebenprodukten bestehen, unter Einstellung eines Molverhältnisses von Salpetersäure zu Toluol von mindestens 1,5:1, vorzugsweise von mindestens 1,8:1, besonders bevorzugt von mindestens 1,9:1 unter adiabatischen Bedingungen zur Reaktion bringt, so daß noch geringe Mengen an Mononitrotoluol nach der Nitrierung in der Reaktionsmischung vorliegen, das den Reaktor kontinuierlich mit einer Temperatur von mindestens 120°C verlassende Reaktionsgemisch durch Entspannungsverdampfung, gegebenenfalls unter gleichzeitiger Wärmezufuhr von mindestens 5 % des Wassers destillativ befreit, gegebenenfalls bis zu 0,6 Mol, vorzugsweise bis zu 0,3 Mol, besonders bevorzugt bis zu 0,2 Mol, 50 bis 100 gew.-%iger Salpetersäure zumischt, anschließend in eine obere Produktphase und eine untere Säurephase auftrennt, die Produktphase aufarbeitet und die Säurephase nach Zugabe von 50 bis 100 gew.-%iger Salpetersäure an den Prozeßanfang zurückführt.

Überraschenderweise wurde gefunden, daß die Kondensation der Brüden vorteilhaft durchgeführt werden kann, wenn man die Reaktion so führt, daß geringe Anteile an MNT (mindestens 5 Gew.-% bezogen auf gebildetes Nitroprodukt) im Reaktionsprodukt verbleiben und die Separation der organischen Phase erst nach der Aufkonzentrierung der Säure erfolgt. Das geringe Mengen an MNT enthaltende Reaktionsprodukt tritt unmittelbar nach Verlassen des Reaktors in die Aufkonzentrierung ein, wobei das vorliegende MNT mit dem Wasser und geringen DNT-Anteilen über Kopf abdestilliert wird. Das Verhältnis von MNT zu DNT im Brüdenkondensat beträgt 10:1 bis 1:5, vorzugsweise 5:1 bis 1:5. Dieses MNT/DNT-Gemisch sorgt dafür, daß das Brüdenkondensat unter den Kondensationsbedingungen des Wassers an den Wärmetauschern flüssig abläuft. Überraschend hierbei ist, daß im Aufkonzentrierschritt nahezu das gesamte MNT mit den Brüden abdestilliert, so daß der im Destillationssumpf verbleibende Anteil an MNT sehr gering ist und die DNT-Ausbeute nur geringfügig gemindert wird.

Das mit den Brüden übergehende MNT/DNT-Gemisch wird abgetrennt und in die Reaktion zurückgeführt.

Dieses Ergebnis ist umso überraschender, da verfahrenstechnische Berechnungen für den Restgehalt an MNT im Sumpf stets deutlich höhere Mengen ergeben.

Völlige MNT-Freiheit des Nitrierproduktes wird erreicht, wenn nach dem Schritt der Aufkonzentrierung vor der Phasentrennung geringe Mengen an 50 bis 100 gew.-%iger Salpetersäure, gegebenenfalls unter gleichzeitigem Einsatz einer Vermischungseinheit, dem Reaktionsgemisch zugegeben werden.

Im Anschluß an den Aufkonzentrierschritt wird die Trennung der Phasen durchgeführt. Diese Phasentrennung kann bei einer deutlich niedrigeren Temperatur stattfinden als bei dem in EP-A 597 361 beanspruchten Verfahren. Dadurch ergeben sich sicherheitstechnische Vorteile. Desweiteren ergeben sich aufgrund der reduzierten thermischen Belastung verminderte Mengen an Nebenprodukten.

Aufgrund des im Reaktionsgemisch erlaubten MNT-Gehaltes sind die Anforderungen an den Reaktor geringer. Dieser muß nicht mehr zwangsläufig auf vollständigen Umsatz ausgelegt sein.

Die zur Kondensation der Brüden erforderliche Restmenge an MNT wird durch die unterstöchiometrische Reaktionsführung oder auch durch Absenken der Säurekonzentration im System erreicht. Über eine verringerte Schwefelsäurekonzentration verringert sich zudem die Löslichkeit der nitrierten Verbindungen und damit die Menge an nitrierten Verbindungen im Säurekreislauf. Desweiteren muß die Säure nach der Reaktion nicht mehr so hoch aufkonzentriert werden.

Bedingt durch die unvollständig geführte Nitrierung kann auch die Menge an Nitriersäure geringer sein, da auf eine absolut vollständige Dinitrierung nicht geachtet werden muß, wodurch u.a. eine geringere Salpetersäurebelastung im Abwasser auftritt.

Ein Molverhältnis von Salpetersäure zu Toluol von über 2,5 ist technisch nicht sinnvoll.

Bei einem Molverhältnis von Salpetersäure zu Toluol von ≥ 2,0 wird die Nitrierung so durchgeführt, daß noch MNT in der Reaktionsmischung vorliegt. Dies kann beispielsweise durch frühzeitigen Abbruch der Reaktion oder unvollständige Vermischung der Reaktionskomponenten erreicht werden.

Die Erfindung soll anhand der nachfolgenden Beispiele näher erläutert werden.

### Beispiel 1

248 g/h (2,692 mol/h) Toluol und 9424 g/h (5,384 mol/h) Nitriersäure (Zusammensetzung: 77,0 Gew.-% H₂SO₄ : 3,6 Gew.-% HNO₃ : 19,4 Gew.-% H₂O) werden jeweils auf 120°C temperiert und in einen Rohrreaktor unter adiabatischen Bedingungen zur Reaktion gebracht. Das mit 160°C den Reaktor verlassende Reaktionsgemisch enthält 7,5 Gew.-% Mononitrotoluol und wird zur Aufkonzentrierung in ein Vakuum von 30 mbar eingebracht. Die Temperatur des Sumpfablaufs wurde variiert. Die über Kopf abgehenden Brüden werden an einem Kühler kondensiert, wobei das Kondensat flüssig abläuft. Der Restgehalt an MNT im so aufkonzentrierten Reaktionsgemisch beträgt bei einer Sumpftemperatur von
110°C 1,05 Gew.-%
115°C 0,44 Gew.-%
120°C 0,21 Gew.-%
125°C 0,17 Gew.-%
130°C 0,10 Gew.-%

### Beispiel 2

261 g/h (2,834 mol/h) Toluol und 9424 g/h (5,384 mol/h) Nitriersäure (Zusammensetzung wie in Beispiel 1) werden jeweils auf 120°C temperiert und in einen Rohrreaktor unter adiabatischen Bedingungen zur Reaktion gebracht. Das mit 165°C den Reaktor verlassende Reaktionsgemisch enthält 13,6 Gew.-% Mononitrotoluol und wird in ein Vakuum von 30 mbar eingebracht. Die Temperatur des Sumpfablaufs bei der Aufkonzentrierung wurde variiert. Die über Kopf abgehenden Brüden werden an einem Kühler kondensiert, wobei das Kondensat flüssig abläuft. Der Restgehalt an MNT im so aufkonzentrierten Reaktionsgemisch beträgt bei einer Sumpftemperatur von
110°C 3,34 Gew.-%
115°C 1,33 Gew.-%
120°C 0,48 Gew.-%
125°C 0,21 Gew.-%
130°C 0,12 Gew.-%

## Patentansprüche

1. Verfahren zur kontinuierlichen Herstellung von Dinitro-lsomerengemischen durch Nitrierung von Toluolen, wobei man Toluol einstufig in einem kontinuierlich betriebenen Reaktor unter Verwendung einer Nitriersäure, bestehend aus (i) 80 bis 100 Gew.-% an anorganischen Bestandteilen, die sich im wesentlichen aus 60 bis 90 Gew.-% Schwefelsäure, 1 bis 20 Gew.-% Salpetersäure und mindestens 5 Gew.-% Wasser zusammensetzen und (ii) 0 bis 20 Gew.-% organischen Bestandteilen, die zu 70 bis 100 % aus Dinitritoluol-lsomeren und zum Rest aus Nebenprodukten bestehen, unter adiabatischen Bedingungen umsetzt, dadurch gekennzeichnet, daß man ein Molverhältnis von Salpetersäure zu Toluol von mindestens 1,5:1 einstellt, so daß das Verhältnis von MNT zu DNT im Brüdenkondensat 10:1 bis 1:5 beträgt **und mindestens 5 Gew.-%, bezogen auf gebildetes Nitroprodukt, an Mononitrotoluol im Reaktionsprodukt verbleiben**, das den Reaktor kontinuierlich mit einer Temperatur von mindestens 120°C verlassende Reaktionsgemisch durch Entspannungsverdampfung, gegebenenfalls unter gleichzeitiger Wärmezufuhr von mindestens 5 % des Wassers destillativ befreit, gegebenenfalls bis zu 0,6 Mol, 50 bis 100 gew.-%ige Salpetersäure zumischt, anschließend in eine obere Produktphase und eine untere Säurephase auftrennt, die Produktphase aufarbeitet und die Säurephase nach Zugabe von 50 bis 100 gew.-%iger Salpetersäure an den Prozeßanfang zurückführt.

## Claims

1. Process for the continuous production of dinitro isomer mixtures by toluene nitration, wherein toluene is reacted under adiabatic conditions in a single stage in a continuously operated reactor using a nitrating acid consisting of (i) from 80 to 100 wt.% inorganic constituents composed substantially of from 60 to 90 wt.% sulfuric acid, from 1 to 20 wt.% nitric acid and at least 5 wt.% water and (ii) from 0 to 20 wt.% organic constituents, consisting of 70 to 100% of dinitrotoluene isomers the remainder being byproducts, characterised in that a molar ratio of nitric acid to toluene of at least 1.5 : 1 is adjusted, such that the ratio of MNT to DNT in the vapour condensate is from 10 : 1 to 1 : 5 and at least 5 wt.% of mononitrotoluene, in relation to nitro product formed, remain in the reaction product, at least 5% of the water is removed by distillation by flash evaporation, optionally with simultaneous supply of heat, from the reaction mixture which leaves the reactor continuously at a temperature of at least 120°C, optionally up to 0.6 mole of 50 to 100 wt.% nitric acid is admixed, followed by separation into an upper product phase and a lower acid phase, the product phase is worked up and the acid phase is returned into the start of the process after the addition of 50 to 100 wt.% nitric acid.

## Revendications

1. Procédé pour la préparation en continu de mélanges d'isomères dinitrés par nitration de toluènes, dans lequel on fait réagir du toluène dans des conditions adiabatiques, en une seule étape, dans un réacteur mis en service en continu, en utilisant un mélange sulfonitrique constitué par (i) des constituants inorganiques à concurrence de 80 à 100% en poids qui sont composés essentiellement d'acide sulfurique à concurrence de 60 à 90% en poids, d'acide nitrique à concurrence de 1 à 20% en poids et d'eau à concurrence d'au moins 5% en poids, et par (ii) des constituants organiques à concurrence de 0 à 20% en poids, qui sont constitués jusqu'à concurrence de 70 à 100% par des isomères du dinitrotoluène et, pour le reste, par des sous-produits, caractérisé en ce qu'on règle un rapport molaire de l'acide nitrique au toluène d'au moins 1,5:1 de telle sorte que le rapport du MNT au DNT dans le condensat de vapeurs s'élève de 10:1 à 1:5 et de telle sorte que du mononitrotoluène subsiste dans le produit réactionnel au moins à concurrence de 5% en poids rapportés au produit nitré obtenu; on libère par distillation le mélange réactionnel quittant le réacteur en continu avec une température d'au moins 120°C, de l'eau à concurrence d'au moins 5%, via évaporation par détente, le cas échéant avec un apport de chaleur simultané; on ajoute par mélange, le cas échéant, de l'acide nitrique à 50 à 100% en poids, jusqu'à concurrence de 0,6 mole; on procède ensuite à une séparation en une phase de produit supérieure et en une phase acide inférieure; on traite la phase de produit; et on renvoie la phase acide au début du procédé après addition d'acide nitrique à 50 à 100% en poids.
